# FASCICULE DE BREVET EUROPEEN

(11) **EP 3 952 739 B1**
(45) Date de publication et mention de la délivrance du brevet: **19.02.2025**
(21) Numéro de dépôt: 20717654.6
(22) Date de dépôt: 08.04.2020
(51) Int. Cl.: A61B 5/11, A61B 5/113, A61B 6/00, A61B 34/00, A61B 34/10, A61B 34/20, A61B 34/30, A61B 17/00, A61B 90/00

(54) **DISPOSITIF ET PROCEDE DE SYNCHRONISATION POUR LA DETERMINATION D'UN INSTANT DU CYCLE RESPIRATOIRE D'UN PATIENT, ET ENSEMBLE COMPORTANT UN ROBOT MEDICAL**
SYNCHRONISATIONSVORRICHTUNG UND VERFAHREN ZUR BESTIMMUNG EINES ZEITPUNKTES DES ATMUNGSZYKLUS EINES PATIENTEN UND ANORDNUNG MIT EINEM MEDIZINISCHEN ROBOTER
SYNCHRONISATION DEVICE AND METHOD FOR DETERMINING AN INSTANT OF THE RESPIRATORY CYCLE OF A PATIENT, AND ASSEMBLY COMPRISING A MEDICAL ROBOT

(30) Priorité: 12.04.2019 FR 1903950
(43) Date de publication de la demande: 16.02.2022
(73) Titulaire: Quantum Surgical, 34000 Montpellier (FR)
(72) Inventeur: LE MEUR, Yann, 78000 Versailles (FR); BLONDEL, Lucien, 34070 Montpellier (FR); BADANO, Fernand, 69006 Lyon (FR); NAHUM, Bertin, 34170 Castelnau-le-Lez (FR)
(74) Mandataire: Ipside
(86) Numéro de dépôt international: PCT/EP2020/060030
(87) Numéro de publication internationale: WO 2020/208078

(56) Documents cités:
- US-A1- 2008 177 280
- US-A1- 2010 067 739
- US-A1- 2018 092 698
- US-A1- 2019 038 365

## Description

### Domaine de l'invention

La présente invention appartient au domaine des interventions médicales mini-invasives, notamment guidées par l'image. La présente invention concerne plus particulièrement un dispositif de synchronisation pour la détermination d'un instant du cycle respiratoire d'un patient pour assister une intervention médicale sur ledit patient et un procédé de détermination d'un instant du cycle respiratoire d'un patient pour assister un geste médical.

### Etat de la technique

Les interventions médicales (diagnostiques, thérapeutiques et/ou chirurgicales) par voie mini-invasive ou percutanée sur un patient deviennent de plus en plus importantes, notamment en oncologie dans les traitements locaux du cancer, en agissant directement sur les cellules de l'organe atteint tel que le foie, le rein, le poumon, le pancréas, le sein, la prostate, etc.

Ces interventions médicales nécessitent généralement l'insertion par un opérateur d'instruments médicaux à l'intérieur du corps du patient jusqu'à une certaine profondeur pour atteindre une zone anatomique cible à traiter.

Afin d'améliorer la précision du geste médical d'insertion, et aussi pour limiter les doses d'irradiation sur le patient et le personnel médical, l'opérateur peut être assisté par un robot médical. Le robot médical permet par exemple, lorsqu'il est associé à une unité de contrôle qui planifie l'intervention sur une image médicale, de positionner un guide d'instrument selon les informations de planification. Dans ce cas, l'insertion de l'instrument doit préférablement être réalisée lorsque le patient est dans les mêmes conditions de respiration que celles dans lesquelles il était lors de l'acquisition de l'image ayant servi à la planification.

En vue de réaliser l'intervention percutanée, un élément appelé « référence patient » comprenant des marqueurs détectables dans une image médicale est préalablement positionné sur le corps du patient, à proximité de la zone anatomique cible. Une image médicale du patient, muni de la référence patient, est acquise par un dispositif d'imagerie (par exemple, un scan CT).

L'intervention médicale est planifiée sur cette image médicale dite intra-opératoire. Alternativement, l'intervention médicale est planifiée sur une image médicale dite pré-opératoire et les données de planification sont recalées à l'image médicale intra-opératoire par recalage d'images au moment de l'intervention.

De manière générale, on considère qu'un instant donné du cycle respiratoire du patient correspond à une position donnée de la zone anatomique cible. Lors de l'acquisition de l'image médicale, le patient se trouve à une certaine phase de son cycle respiratoire. Au moment de l'insertion de l'instrument (postérieurement à l'acquisition d'image), il est nécessaire que le patient se trouve dans la même phase de cycle respiratoire, de sorte que les positions des organes du patient correspondent aux positions de ces mêmes organes lors de l'acquisition de l'image médicale. Si l'insertion de l'instrument médical est effectuée à une autre phase du cycle respiratoire du patient, l'anatomie d'intérêt peut avoir bougée du fait de la respiration et la zone anatomique cible ne pourra donc pas être atteinte avec précision.

Le document EP1123138 décrit un dispositif d'imagerie couplé à un système délivrant un traitement de radiothérapie en fonction des mouvements d'un patient mesurés par un capteur et une caméra. Le traitement de radiothérapie n'est délivré que lorsque les mouvements du patient ne dépassent pas un certain seuil. Cependant, il est nécessaire d'imager le patient en permanence. Le système est de plus très dépendant du dispositif d'imagerie utilisé.

Le document US 7920909 décrit une méthode de détermination a posteriori de l'instant du cycle respiratoire auquel une image médicale est prise par comparaison avec une série d'images médicales du même patient prises à différents instants du cycle respiratoire du patient. Cette méthode nécessite d'avoir un nombre important d'images médicales du patient. Par ailleurs, l'instant exact du cycle respiratoire du patient ne peut être déterminé avec précision que si l'on dispose d'un grand nombre d'images médicales du patient de manière à échantillonner assez finement le cycle respiratoire. Cette méthode implique également que le cycle respiratoire du patient soit régulier.

Le document WO2019026089 décrit une ceinture entourant l'abdomen d'un patient muni d'un premier capteur indiquant la taille de l'abdomen durant l'acquisition d'image et un second capteur indiquant la position du patient durant l'acquisition d'image. Ces deux capteurs enregistrent la position d'inhalation maximale du patient lorsqu'il respire. Lors de l'acquisition d'image et de l'intervention médicale, le patient doit bloquer sa respiration lorsque cette position d'inhalation maximale est atteinte. Ce dispositif ne donne pas d'indication précise quant au moment exact du cycle respiratoire lors de l'acquisition de l'image médicale, est très dépendant du patient. De plus, le cycle respiratoire du patient est considéré comme régulier.

Le document US 2010/067739 décrit un dispositif pour déterminer la position d'une cible à partir d'une technique d'imagerie. US 2010/067739 propose d'associer des images à un état respiratoire (expiration, inspiration, blocage de la respiratoire lors de l'expiration ou lors de l'inspiration) afin de reconstituer l'ordre des images acquises à différents moments du cycle respiratoire.

### Exposé de l'invention

La présente invention vise à remédier à tout ou partie des limitations des solutions de l'art antérieur, notamment celles exposées ci-avant.

A cet effet, il est proposé par la présente invention un dispositif de synchronisation pour la détermination d'un instant du cycle respiratoire d'un patient pour assister une intervention médicale sur ledit patient tel que défini dans la revendication 1.

Un tel dispositif de synchronisation présente l'avantage de n'être lié à aucun dispositif d'imagerie par rayons X particulier. Tout type de dispositif d'imagerie par rayons X existant peut ainsi être associé au dispositif de synchronisation.

Un tel dispositif de synchronisation permet avantageusement la détermination précise de la phase du cycle respiratoire lors de l'acquisition de l'image médicale, sans considérer que le cycle respiratoire est régulier.

Un tel dispositif de synchronisation permet également d'assurer la précision du geste médical dans la mesure où la phase du cycle respiratoire du patient est connue avec précision lors de l'acquisition de l'image médicale et peut être reproduite et contrôlée lors du geste médical.

Dans des modes particuliers de réalisation, le dispositif de localisation peut en outre comporter l'une ou plusieurs des caractéristiques décrites ci-après.

Selon l'invention, le dispositif de localisation est configuré pour enregistrer, en continu, des mouvements de la référence patient, les mouvements de la référence patient correspondant au cycle respiratoire du patient, et pour transmettre à l'unité de contrôle. L'unité de contrôle est configurée pour :
- déterminer une position, dite cible, de la référence patient dans un repère du dispositif de localisation, à partir du au moins un élément de localisation et du détecteur de rayons X positionnés sur la référence patient,
- déterminer une position, dite candidate, de la référence patient, dans le repère du dispositif de localisation, à partir du au moins un élément de localisation positionné sur la référence patient,
- comparer la position candidate et la position cible,
- lorsque la position candidate ne se situe pas dans une plage de tolérance prédéfinie par rapport à la position cible, réitérer les étapes de détermination de la position candidate et de comparaison des positions cible et candidate jusqu'à ce que la position candidate se situe dans une plage de tolérance prédéfinie par rapport à la position cible.

Dans des modes particuliers de réalisation, l'unité de contrôle est configurée pour déterminer, à partir de l'ensemble des positions prises par la référence patient pendant la durée de l'exposition aux rayons X, la position cible de la référence patient.

Dans un exemple particulier de réalisation, l'unité de contrôle est configurée pour déterminer la position cible à partir du calcul d'une moyenne de l'ensemble des positions prises par la référence patient pendant la durée de l'exposition aux rayons X.

Dans un autre exemple de réalisation, l'unité de contrôle est configurée pour déterminer la position cible à partir du calcul d'une moyenne de l'ensemble des positions prises par la référence patient pendant la durée de l'exposition aux rayons X. pondéré par la dose de rayons X reçue à chaque position.

Dans des modes particuliers de réalisation, l'unité de contrôle est configurée pour estimer, à partir d'un modèle de prédiction du cycle respiratoire du patient, l'instant où la référence patient est proche de la position cible, lors de la détermination d'une position candidate.

Dans des modes particuliers de réalisation, l'unité de contrôle est configurée pour estimer, à partir d'un modèle de prédiction du cycle respiratoire du patient, le moment du blocage de la respiration du patient lors de la détermination d'une position candidate.

Dans des modes particuliers de réalisation, l'unité de contrôle est configurée pour planifier une intervention assistée par un robot médical entre les étapes de détermination de la position cible et de la position candidate.

Dans des modes particuliers de réalisation, le dispositif de localisation est un dispositif de localisation optique et dans lequel la référence patient comporte au moins trois éléments de localisation optiques.

Dans des exemples particuliers de réalisation, le dispositif de localisation optique est une caméra stéréoscopique infrarouge et les éléments de localisation optiques sont des sphères réfléchissantes.

Dans des modes particuliers de réalisation, le dispositif de localisation est un dispositif de localisation électromagnétique et dans lequel la référence patient comporte au moins un élément de localisation électromagnétique.

Dans des exemples particuliers de réalisation, le dispositif de localisation électromagnétique est un générateur de champ électromagnétique et le au moins un élément de localisation électromagnétique comporte au moins deux bobines conductrices.

Dans des modes particuliers de réalisation, le détecteur de rayons X est un dosimètre ou un scintillateur.

Dans des modes particuliers de réalisation, le dispositif de synchronisation comporte un équipement d'interface homme-machine configuré pour informer un opérateur, sous la forme d'un signal sonore et/ou d'un signal visuel, du résultat de la comparaison entre la position candidate et la position cible. L'opérateur est alors informé, selon le signal reçu, si le moment est optimal, ou non, pour pratiquer le geste médical souhaité.

Dans des exemples particuliers de réalisation, l'équipement d'interface homme-machine est un écran de visualisation.

L'invention est également relative à un ensemble comportant un robot médical et le dispositif de synchronisation selon au moins l'un de ses modes de réalisation, le robot médical comportant une base, un bras articulé dont une extrémité est reliée à la base, et une unité de contrôle. Le robot médical permet avantageusement de pratiquer le geste médical à la place de l'opérateur.

Dans des modes particuliers de réalisation, l'ensemble peut en outre comporter l'une ou plusieurs des caractéristiques décrites ci-après.

Dans des exemples particuliers de réalisation, l'unité de contrôle du robot médical et l'unité de contrôle du dispositif de synchronisation forment une seule et même unité de contrôle.

Dans des exemples particuliers de réalisation, le robot médical comporte un équipement d'interface homme-machine.

L'invention est également relative à un procédé de détermination d'un instant du cycle respiratoire d'un patient, pour assister une intervention médicale sur ledit patient planifiée, tel que défini dans la revendication 13. Le procédé selon l'invention permet par exemple d'assister un opérateur à pratiquer un geste médical sur le patient.

L'étape 101 d'acquisition d'une image médicale peut être réalisée pendant un blocage de la respiration du patient. Le blocage de la respiration du patient est temporaire, la respiration du patient est débloquée une fois l'image médicale acquise.

Alternativement, cette étape 101 d'acquisition d'une image médicale peut être réalisée sans blocage de la respiration du patient.

Une telle image médicale obtenue lors de cette étape 101, sur laquelle la zone anatomique cible à traiter est visible, est notamment destinée à permettre à l'opérateur de planifier un geste médical, réalisé par lui-même ou par un robot médical l'assistant pour une intervention médicale.

La position cible de la référence patient est déterminée lors de l'acquisition de l'image médicale, à partir des mouvements du au moins un élément de localisation configuré pour être détecté par un dispositif de localisation et à partir des données obtenues par le détecteur de rayons X.

Dans un exemple de mise en œuvre de l'étape 103 de détermination de la position candidate, une fois la position cible de la référence patient déterminée, et la planification du geste médical organisée, la respiration du patient est à nouveau bloquée. Ce blocage de la respiration du patient est temporaire, le temps de déterminer la position, nommée position candidate, de la référence patient et de comparer cette position candidate avec la position cible de la référence patient, pendant ce moment de blocage de la respiration, afin de déterminer si la respiration du patient a été bloquée à une même phase du cycle respiratoire que lors de l'acquisition de l'image médicale ayant servi à la planification.

Dans un autre exemple de mise en œuvre de l'étape 103 de détermination de la position candidate, une fois la position cible de la référence patient déterminée, et la planification du geste médical organisée, la position candidate est déterminée sans blocage de la respiration du patient. Le moment où la position candidate se situe à proximité de la position cible peut par exemple être estimé à partir d'un modèle de prédiction du cycle respiratoire du patient.

Quel que soit l'exemple de mise en œuvre de l'étape 103, le geste médical peut être autorisé et pratiqué uniquement lorsque le patient se retrouve dans les mêmes conditions de respiration.

Un tel procédé permet de déterminer le moment optimal où l'opérateur peut pratiquer son geste médical. Un tel procédé permet ainsi avantageusement d'améliorer la précision du geste médical d'insertion, car le geste médical est réalisé lorsque le patient est dans les mêmes conditions de respiration que celles dans lesquelles il était lors de l'acquisition de l'image médicale ayant servi à la planification.

Le procédé selon l'invention ne nécessite pas comme prérequis que le cycle respiratoire est régulier.

Un tel procédé permet également avantageusement de limiter les doses d'irradiation sur le patient ainsi que sur le personnel médical, l'acquisition d'une seule image médicale étant suffisante pour mettre en œuvre le procédé selon l'invention.

Le procédé selon l'invention peut en outre répondre à l'une ou plusieurs des caractéristiques décrites ci-après, mises en œuvre isolément ou en chacune de leurs combinaisons techniquement opérantes.

Dans des modes particuliers de mise en œuvre, lorsque la position candidate de la référence patient ne se situe pas dans une plage de tolérance prédéfinie par rapport à la position cible, les étapes 103 et 104 sont réitérées jusqu'à ce que la position candidate de la référence patient se situe dans une plage de tolérance prédéfinie par rapport à la position cible.

De préférence, la plage de tolérance est de ± 10% de la position cible.

Dans des modes particuliers de mise en œuvre, la position cible de la référence patient est déterminée à partir de l'ensemble des positions prises par la référence patient pendant la durée de l'exposition aux rayons X, c'est à dire pendant la durée de l'acquisition de l'image médicale.

Par exemple, la position cible est déterminée à partir du calcul d'une moyenne de l'ensemble des positions prises par la référence patient pendant la durée de l'exposition aux rayons X.

Dans des modes particuliers de mise en œuvre, le moment où la position candidate se situe dans une plage de tolérance prédéfinie par rapport à la position cible est estimé à partir d'un modèle de prédiction du cycle respiratoire du patient.

Dans des modes particuliers de mise en œuvre, notamment pour anticiper le moment où la respiration du patient doit être temporairement bloquée à l'étape 103, le moment du blocage de la respiration du patient est estimé à partir d'un modèle de prédiction du cycle respiratoire du patient.

Dans des modes particuliers de mise en œuvre, un signal sonore et/ou un signal visuel est généré selon le résultat de la comparaison entre la position candidate et la position cible de la référence patient. Un tel signal permet avantageusement d'alerter l'opérateur s'il peut ou non pratiquer le geste médical.

Dans des modes particuliers de mise en œuvre, l'étape de planification d'une intervention, notamment assistée par un robot médical, est réalisée entre les étapes 102 de détermination de la position cible et 103 de détermination de la position candidate.

### Présentations des figures

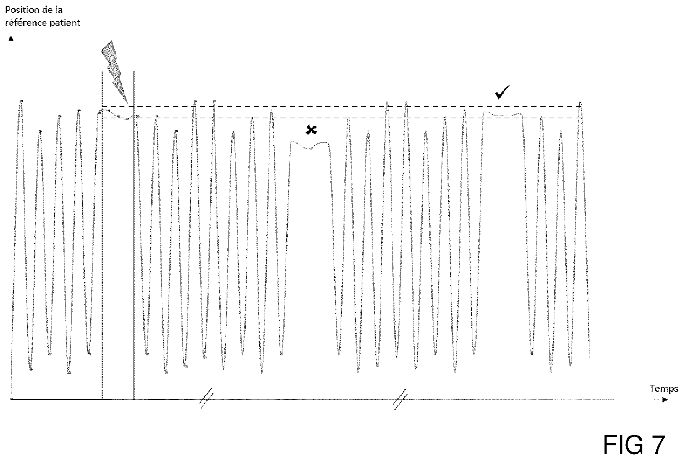

Les caractéristiques et avantages de l'invention apparaîtront plus clairement à la lumière des exemples de mise en œuvre ci-après, fournis à simple titre illustratif et nullement limitatifs de l'invention, avec l'appui des figures 1 à 7, dans lesquelles :
[Fig. 1] La figure 1 illustre une représentation schématique d'un dispositif de synchronisation selon l'invention et d'un robot médical,
[Fig. 2] La figure 2 illustre un exemple de réalisation d'une référence patient,
[Fig. 3] La figure 3 illustre un autre exemple de réalisation d'une référence patient du dispositif de synchronisation,
[Fig. 4] La figure 4 représente un enregistrement, par une caméra, des mouvements dans le temps d'une référence patient positionnée sur le patient,
[Fig. 5] La figure 5 représente une courbe illustrant les mouvements dans le temps d'une référence patient positionnée sur le patient,
[Fig. 6] La figure 6 représente une courbe illustrant les mouvements dans le temps d'une référence patient positionnée sur le patient, lors de l'acquisition d'une image médicale,
[Fig. 7] La figure 7 représente une courbe illustrant les différentes étapes du procédé selon l'invention.

### Description détaillée d'un mode de réalisation de l'invention

L'invention est relative à un procédé de détermination d'un instant du cycle respiratoire d'un patient pour assister une intervention médicale, notamment pour guider un opérateur lors de la réalisation un geste médical. L'intervention médicale est par exemple de type mini-invasive, au niveau d'une zone anatomique cible du corps d'un patient.

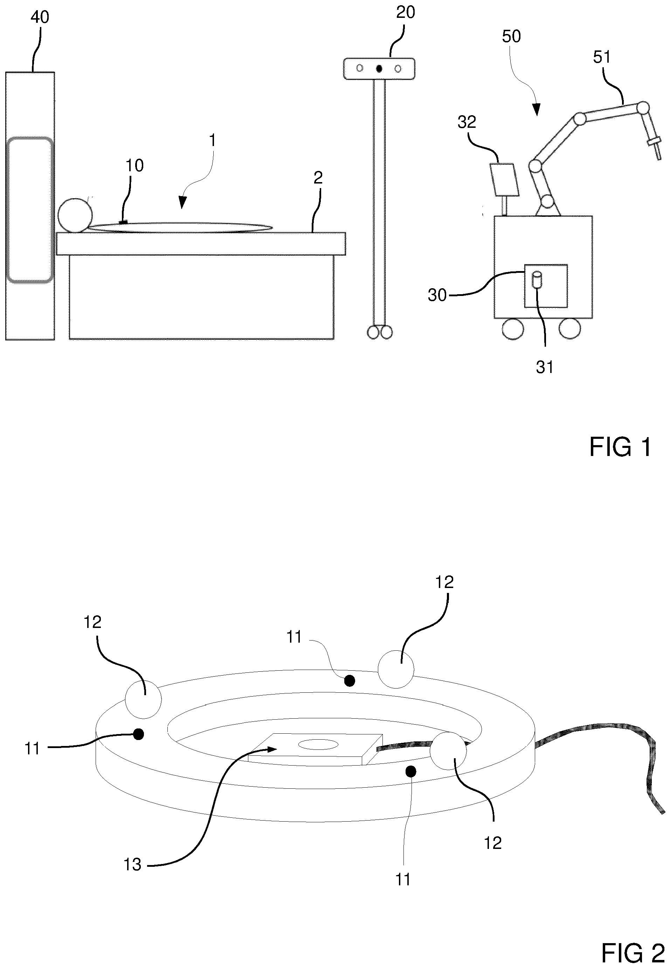

Un exemple de réalisation d'un dispositif apte à mettre en œuvre le procédé selon l'invention est préalablement décrit et illustré schématiquement sur la figure 1. La figure 1 illustre également le patient 1 allongé sur une table d'intervention 2.

Le dispositif de synchronisation est préférentiellement adapté pour être associé à un dispositif d'imagerie 40 par rayons X, comme illustré sur la figure 1.

De manière connue, le dispositif d'imagerie 40 par rayons X est configuré pour acquérir une image médicale du patient dans laquelle, notamment, la zone anatomique cible à traiter est visible. Cette image médicale permet également de planifier le geste de l'opérateur ou d'un robot médical assistant l'opérateur pour une intervention médicale. Elle peut être utilisée pour déterminer la position que doit prendre un instrument médical par rapport à la zone anatomique cible. Le dispositif de synchronisation selon l'invention peut avantageusement fonctionner avec tout type de dispositif d'imagerie médicale 40 par rayons X.

Dans des exemples non limitatifs de dispositifs d'imagerie 40 par rayons X, on peut citer notamment le scanner CT, ou encore l'imageur CBCT (acronyme de « Cone Beam Computed Tomography » pour « tomographie calculée à faisceau conique »).

De manière préférée également, le dispositif de synchronisation est adapté pour être associé à un robot médical 50, comme illustré sur la figure 1. Le robot médical 50 est configuré pour assister un geste de l'opérateur lors de l'intervention médicale. Un tel robot médical permet entre autre d'aider le praticien à positionner, maintenir ou guider un instrument médical. Un exemple de robot médical sera décrit ultérieurement, à titre illustratif uniquement.

Le dispositif de synchronisation comporte :
- un élément, nommé par la suite référence patient 10, destiné à être positionné sur le patient 1,
- un dispositif de localisation 20,
- une unité de contrôle 30 configurée pour recevoir et traiter les données provenant du dispositif de localisation 20 et celles provenant de la référence patient 10.

L'unité de contrôle 30 est également configurée pour recevoir, via un moyen de communication filaire ou non, l'image médicale acquise par le dispositif d'imagerie 40 par rayons X et la stocker.

La référence patient 10 est destinée à être positionnée sur le corps du patient 1, à proximité de la zone anatomique cible du patient. La référence patient 10 est positionnée sur le corps du patient de telle sorte que ses mouvements suivent les mouvements respiratoires du patient.

La référence patient 10 est configurée d'une part pour être visible par le dispositif de localisation 20, et d'autre part pour être détectable dans une image médicale du patient acquise par le dispositif d'imagerie 40 par rayons X.

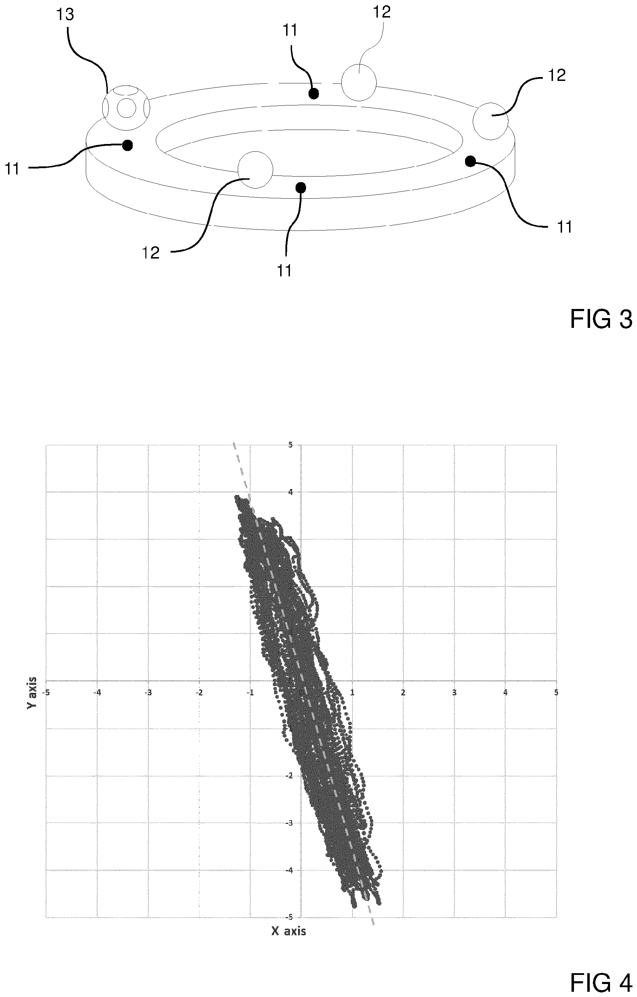

La référence patient 10 comporte avantageusement des marqueurs radio-opaques 11, comme illustré sur les figures 2 et 3, configurés pour être détectables dans l'image médicale. De préférence, les marqueurs radio-opaques 11 sont au nombre d'au moins trois. La géométrie et les positions respectives desdits marqueurs radio-opaques 11 sont connues afin de pouvoir déterminer avec précision la position de la référence patient 10 par rapport à la zone anatomique cible sur l'image médicale.

Dans des exemples préférés de réalisation de marqueurs radio-opaques 11, lesdits marqueurs radio-opaques 11 sont des billes en céramique radio-opaques ou encore des billes métalliques radio-opaques.

La référence patient 10 comporte en outre au moins un élément de localisation 12, comme illustré sur les figures 2 et 3, configuré pour être visible par le dispositif de localisation 20. Le au moins un élément de localisation 12 coopère avec le dispositif de localisation 20.

Le type du au moins un élément de localisation 12 sur la référence patient 10 dépend du dispositif de localisation 20 utilisé. Le au moins un élément de localisation 12 sera décrit plus précisément ultérieurement, en relation avec le dispositif de localisation 20 associé.

La référence patient 10 comporte en outre un détecteur 13 de rayons X, comme illustrés sur les figures 2 ou 3. Le détecteur 13 de rayons X est destiné à coopérer avec le dispositif d'imagerie 40par rayons X. Le détecteur 13 de rayons X est configuré pour détecter le moment où l'émission de rayons X a eu lieu, c'est-à-dire le moment où l'image médicale a été prise.

Dans un exemple de réalisation, comme illustré sur la figure 2, le détecteur 13 de rayons X est un dosimètre. Par dosimètre, on entend un capteur apte à mesurer une dose d'irradiation. Le dosimètre permet notamment de détecter la présence ou l'absence de rayons X. Le dosimètre peut également indiquer la dose de rayons X reçus dans le temps. Dans un exemple de réalisation, le dosimètre est un dosimètre de type photodiode PIN en silicium sensible aux rayons X. Le dosimètre est configuré pour transmettre, via un moyen de communication filaire, des données relatives à la présence ou à l'absence de rayons X, ainsi que la dose de rayons X reçus dans le temps, à l'unité de contrôle 30.

Dans un autre exemple de réalisation, comme illustré sur la figure 3, le détecteur 13 de rayons X est un scintillateur. Par scintillateur, on entend un matériau qui absorbe les rayons X, émis par le dispositif d'imagerie 40 par rayons X, et qui émet un signal lumineux pouvant être détecté par une caméra. Dans un exemple de scintillateur, le scintillateur est un scintillateur inorganique de type Csl(Tl) (Iodure de césium dopé au thallium). La caméra est configurée pour transmettre des données relatives à la présence ou à l'absence de rayons X dans le temps, à l'unité de contrôle 30.

L'unité de contrôle 30 reçoit et stocke les données transmises par le détecteur de rayons X.

Le dispositif de localisation 20 est configuré pour acquérir des données relatives à la position dans le temps de la référence patient 10, dans un repère dudit dispositif de localisation.

Les mouvements de la référence patient 10, dus aux mouvements de respiration du patient 1, sont suivis et enregistrés par le dispositif de localisation 20 grâce à au moins un élément de localisation 12 positionné sur ladite référence patient. Dans un mode de réalisation, le dispositif de localisation est un dispositif de localisation optique, de préférence une caméra 20.

Dans des exemples non limitatifs, la caméra peut être :
- une caméra stéréoscopique infrarouge,
- une caméra à lumière structurée,
- une caméra à mesure de temps de vol (« Time of Flight camera » ou « ToF camera » dans la littérature anglo-saxonne),
- une caméra à mesure de profondeur (par exemple une caméra RGB-D), etc. La caméra 20 est couplée préférentiellement avec au moins trois éléments de localisation optiques positionnés sur la référence patient 10.

Dans une forme de réalisation, les éléments de localisation optiques sont des marqueurs passifs, tels que par exemple des sphères réfléchissantes.

Dans une autre forme de réalisation, les éléments de localisation optiques sont des marqueurs actifs, tels que par exemple des éléments émettant de la lumière infrarouge.

Dans un exemple de réalisation, lorsque la caméra 20 est une caméra stéréoscopique infrarouge, les éléments de localisation 12 optiques sont des sphères réfléchissantes.

Les éléments de localisation optiques sont de dimensions connues et sont placés sur la référence patient 10 selon une géométrie connue.

Lorsqu'un robot médical 50 est utilisé pour assister l'opérateur lors d'une intervention médicale, des éléments de localisation optiques, de préférence de même type que ceux positionnés sur la référence patient, sont positionnés sur le robot médical.

La caméra 20 est disposée de sorte que les éléments de localisation 12 optiques de la référence patient 10, le cas échéant ceux du robot médical, se situent dans une zone de mesure de ladite caméra.

La caméra 20 est configurée pour réaliser en continu des acquisitions d'images, lesdites images contenant les informations sur les coordonnées des éléments de localisation 12 optiques, donc les coordonnées de la référence patient et, le cas échéant, celles du robot médical. Les coordonnées de la référence patient sont transmises en continu à l'unité de contrôle 30 pour y être traitées et pour déterminer les positions de la référence patient dans le temps, et le cas échéant celles du robot.

Les éléments de localisation 12 optiques et le dispositif de localisation optique forment un système de navigation optique.

Dans un autre mode de réalisation, le dispositif de localisation est un dispositif de localisation électromagnétique, qui comporte un générateur de champ électromagnétique.

Le générateur de champ électromagnétique est couplé préférentiellement avec au moins un élément de localisation 12 électromagnétique, dit capteur électromagnétique, positionné sur la référence patient 10.

Le au moins un capteur électromagnétique comporte par exemple au moins deux bobines conductrices qui peuvent être configurées pour mesurer six degrés de liberté lorsque ledit au moins un capteur électromagnétique est soumis à un champ électromagnétique externe. Chaque bobine du au moins un capteur électromagnétique produit un signal électrique induit ayant des caractéristiques qui dépendent de la position et de l'orientation de la bobine par rapport au champ électromagnétique.

Les bobines du au moins un capteur électromagnétique sont placées sur la référence patient 10 selon une géométrie connue.

Le générateur de champ électromagnétique est disposé de sorte que le au moins un capteur électromagnétique de la référence patient 10 se situe dans une zone de mesure dudit générateur de champ électromagnétique.

Le générateur de champ électromagnétique et le au moins un capteur 12 électromagnétique permettent d'acquérir en continu, des données sur la position du au moins un capteur 12 électromagnétique de la référence patient.

Les données acquises par le au moins un capteur 12 électromagnétique sont transmises à l'unité de contrôle 30 pour y être traitées et déterminer la position de la référence patient 10.

Un ou plusieurs capteurs électromagnétiques peuvent également être positionnés sur le robot médical, lorsqu'il est envisagé de recourir à un tel robot médical pour l'intervention médicale. Ce ou ces capteurs électromagnétiques sont de préférence du même type que ceux positionnés sur la référence patient 10. Les données acquises par ce ou ces capteurs électromagnétiques sont également transmises à l'unité de contrôle 30 pour y être traitées et déterminer la position du robot médical.

Le ou les capteurs électromagnétiques et le dispositif de localisation électromagnétique forment un système de navigation électromagnétique.

Lorsque le dispositif de localisation électromagnétique est choisi, aucune caméra n'est nécessaire. Il est ainsi préférable d'utiliser un dosimètre, plutôt qu'un scintillateur (qui nécessite une caméra), comme détecteur de rayons X.

L'unité de contrôle 30 reçoit et enregistre simultanément les données transmises par le dispositif de localisation, et celles de la référence patient, ainsi que les données, transmises par le détecteur de rayons X, relatives à la dose de rayons X reçue par la référence patient dans le temps ou à la présence/absence de rayons X. L'unité de contrôle 30 est configurée pour déterminer les positions de la référence patient 10, notamment lors de l'acquisition de l'image médicale (dans le cas où la respiration du patient est bloquée et dans le cas où la respiration du patient n'est pas bloquée), comme il sera décrit ultérieurement.

L'unité de contrôle 30 comporte préférentiellement un module mémoire 31 dans lequel est enregistré notamment l'image médicale et les différentes positions de la référence patient dans le temps.

Le dispositif de synchronisation comporte de préférence un équipement d'interface homme-machine 32. Dans un exemple de réalisation, l'équipement d'interface homme-machine est un écran de visualisation, de préférence tactile. L'équipement d'interface homme-machine 32 peut être utilisé pour afficher l'image médicale ou encore l'évolution, dans le temps, de la position de la référence patient 10.

Le dispositif de synchronisation peut comporter un voyant lumineux, par exemple une diode électroluminescente (DEL). Ledit voyant lumineux peut être positionné à proximité de l'équipement d'interface homme-machine. Le voyant lumineux du dispositif de synchronisation est avantageusement configuré pour transmettre une information (ou signal) visuelle à l'opérateur au moment de l'intervention médicale.

Le dispositif de synchronisation peut comporter une alarme. Ladite alarme est avantageusement configurée pour transmettre une information (ou signal) sonore à l'opérateur au moment de l'intervention médicale.

Comme décrit antérieurement, le dispositif de synchronisation peut également être associé à un robot médical.

La figure 1 illustre un exemple de robot médical. Le robot médical comporte une base. La base du robot médical peut être équipée de roues. Comme illustré à la figure 1, ce qui permet au robot médical de se déplacer selon différentes directions par des mouvements de translation et/ou de rotation. Le robot médical comporte un bras articulé 51 dont une extrémité est reliée à la base. Un instrument médical peut être monté sur un porte-outil à l'extrémité libre, dite extrémité distale, du bras articulé 51. Le bras articulé 51 comporte de préférence au moins six degrés de liberté pour pouvoir positionner et/ou déplacer l'instrument médical dans un espace à trois dimensions. Le bras articulé peut comporter par exemple une ou plusieurs articulations.

Le bras articulé 51 est contrôlé par une unité de contrôle du robot médical.

Dans un mode de réalisation, l'unité de contrôle 30 du dispositif de synchronisation et l'unité de contrôle du robot médical sont distinctes mais liées entre elles.

Dans un autre mode de réalisation, comme illustré sur la figure 1, l'unité de contrôle 30 du dispositif de synchronisation et l'unité de contrôle du robot médical forment une seule et même unité de contrôle.

Le robot médical 50 peut comporter un équipement d'interface homme-machine pour permettre à l'opérateur de contrôler le dispositif robotisé et, éventuellement, de visualiser des images en relation avec le geste médical à réaliser.

Dans un mode préféré de réalisation, l'équipement d'interface homme-machine 32 du dispositif de synchronisation et l'équipement d'interface homme-machine du robot médical forment un seul et même équipement d'interface homme-machine.

Le robot médical peut également comporter un voyant lumineux, par exemple une diode électroluminescente (DEL). Ledit voyant lumineux peut être positionné sur le bras articulé, par exemple à son extrémité distale. Ledit voyant lumineux du robot médical est configuré pour transmettre une information visuelle à l'opérateur au moment de l'intervention médicale.

Dans un mode préféré de réalisation, le voyant lumineux du dispositif de synchronisation et le voyant lumineux du robot médical forment un seul et même voyant lumineux.

Le robot médical peut également comporter une alarme. Ladite alarme est avantageusement configurée pour transmettre une information (ou signal) sonore à l'opérateur au moment de l'intervention médicale.

Dans un mode préféré de réalisation, l'alarme du dispositif de synchronisation et l'alarme du robot médical forment une seule et même alarme.

Le dispositif de synchronisation et le robot médical forment un ensemble. L'ensemble peut également comporter le dispositif d'imagerie 40 par rayons X. Le procédé pour déterminer un instant du cycle respiratoire du patient en vue d'assister une intervention médicale selon l'invention est à présent décrit. Un tel procédé a pour but d'informer un opérateur lors d'une intervention médicale, du moment optimal où il peut pratiquer son geste médical, tel que l'insertion d'un instrument médical dans le corps du patient 1.

Il est clair que le geste médical en tant que tel, par exemple l'insertion de l'aiguille dans la zone anatomique cible du patient 1, n'est exécuté par l'opérateur qu'ultérieurement au procédé selon l'invention. L'exécution d'un tel geste médical ne fait donc pas partie du procédé selon l'invention.

Le jour programmé de l'intervention, préalablement à l'exécution du procédé, la référence patient 10 est positionnée sur le corps du patient, au niveau de la zone anatomique cible à traiter du patient 1. Le patient est ensuite, ou préalablement, installé sur la table d'intervention. On considère que les mouvements de la référence patient 10 correspondent au cycle respiratoire du patient.

Le procédé sera décrit dans le cas d'un dispositif de localisation optique, en l'occurrence une caméra. On considère que la caméra 20 est installée par rapport au patient de sorte que la référence patient 10 est située dans sa zone de mesure.

Enfin, dans le cas où l'opérateur a recours à un robot médical pour l'assister, on considère que le robot médical est placé à proximité du patient, à une position permettant au bras articulé dudit robot médical de réaliser l'ensemble des actions à effectuer sur la zone anatomique cible du patient.

Dans une première étape 100, les mouvements de la référence patient 10 sont enregistrés en continu.

Les mouvements de la référence patient sont visualisés par la caméra.

Dans un exemple de mise en œuvre, la caméra 20 acquiert en continu des images de sa zone de mesure et détermine, pour chaque image, les coordonnées de la référence patient, à partir des éléments de localisation 12 optiques placés sur la référence patient 10.

La caméra transmet, en continu, les coordonnées de la référence patient à l'unité de contrôle 30 pour traitement et enregistrement dans son module mémoire 31. La caméra acquiert les images préférentiellement au moins pendant toute la durée du procédé selon l'invention.

Les coordonnées de la référence patient dans chaque image correspondent à la position de la référence patient, dans le repère de la caméra.

Dans une deuxième étape 101, une image médicale, dite intra-opératoire, de la zone anatomique cible du patient est obtenue.

Dans un premier mode de mise en œuvre, la respiration du patient a été bloquée pendant l'obtention de l'image médicale.

Dans une première phase de ce premier mode, la respiration du patient est bloquée.

Dans un exemple de mise en œuvre de cette première phase, lorsque le patient est sous anesthésie générale, le respirateur d'un dispositif d'anesthésie est temporairement débranché, par exemple en fin d'inspiration ou en fin d'expiration. Le patient est plongé dans une apnée dite contrôlée.

Dans un autre exemple de mise en œuvre de cette première phase, lorsque le patient est sous anesthésie locale, il est demandé au patient de se mettre en apnée volontaire, par exemple en fin d'inspiration ou en fin d'expiration.

Dans une deuxième phase de ce premier mode, on acquiert une image médicale intra-opératoire du patient.

L'image médicale intra-opératoire est prise au moyen du dispositif d'imagerie 40 par rayons X. Le dispositif d'imagerie 40 par rayons X, par exemple un CT-scan, est activé et émet des rayons X en direction du patient, notamment la zone anatomique cible.

L'image médicale intra-opératoire provenant du dispositif d'imagerie par rayons X est transmise et stockée dans le module mémoire 31 de l'unité de contrôle 30 du dispositif de synchronisation. L'image médicale intra-opératoire est également affichée sur l'équipement d'interface homme-machine 32.

On peut distinguer sur cette image médicale intra-opératoire la référence patient 10, via la détection des marqueurs radio opaques 11, et la zone anatomique cible.

Pendant l'émission des rayons X par le dispositif d'imagerie 40 par rayons X, lorsque la référence patient 10 du dispositif de synchronisation comporte un scintillateur, lesdits rayons X émis par ledit dispositif d'imagerie 40 par rayons X sont absorbés par ledit scintillateur. En réponse à cette absorption, le scintillateur émet un signal lumineux détecté par la caméra.

Les données relatives à l'absence ou la présence de rayons X sont transmises par la caméra 20, à l'unité de contrôle 30.

Dans une variante de mise en œuvre, pendant l'émission des rayons X par le dispositif d'imagerie 40 par rayons X, lorsque la référence patient 10 du dispositif de synchronisation comporte un dosimètre, les rayons X émis par ledit dispositif d'imagerie 40 par rayons X sont absorbés par ledit dosimètre. En réponse à cette absorption, le dosimètre enregistre la dose de rayons X qu'il absorbe.

Les données relatives à la dose de rayons X reçue par unité de temps par le dosimètre sont transmises par le dosimètre à l'unité de contrôle 30.

Dans une troisième phase de ce premier mode de mise en œuvre, après l'acquisition de l'image médicale intra-opératoire, la respiration du patient est débloquée.

Dans un exemple de mise en œuvre de cette troisième phase, lorsque le patient est sous anesthésie générale, le respirateur est rebranché.

Dans un autre exemple de mise en œuvre de cette troisième phase, lorsque le patient est sous anesthésie locale, il est demandé au patient de respirer à nouveau.

Dans un deuxième mode de mise en œuvre, la respiration du patient n'a pas été bloquée pendant l'obtention de l'image médicale.

Dans ce cas, l'acquisition de l'image est réalisée de manière équivalente à celle de la deuxième phase du premier mode de réalisation. Le patient respire normalement pendant l'acquisition de l'image médicale.

Dans une troisième étape 102 du procédé, une position cible de la référence patient 10 lors de l'acquisition de l'image médicale intra-opératoire est déterminée.

L'unité de contrôle 30 reçoit d'une part, en continu, les coordonnées de la référence patient dans le repère de la caméra, et peut en déduire les positions dans le temps de la référence patient, et donc le cycle respiratoire du patient.

Les coordonnées de la référence patient 10, donc sa position, sont obtenues dans le repère de la caméra. Or, la référence patient, en fonction de son orientation par rapport au sol, ne suit pas toujours un mouvement vertical perpendiculaire au sol. De plus, la caméra peut avoir été orientée de telle sorte que son axe X ne soit pas toujours perpendiculaire au sol. Le mouvement de la référence patient peut alors être représenté par une composante sur chacun des axes de la caméra.

La figure 4 illustre, à titre d'exemple, un enregistrement réel, pendant une minute, par une caméra, des mouvements d'une référence patient sur un patient. Chaque point correspond à une position dans le temps de la référence patient, dans un plan XY. On constate, dans cet exemple, que le mouvement de la référence patient s'effectue selon l'axe tracé en pointillé sur la figure 4.

Pour une meilleure interprétation du mouvement de la référence patient, et par analogie du cycle respiratoire du patient, il est préférable d'obtenir une courbe à une dimension, illustrant le mouvement oscillatoire de la référence patient, donc du cycle respiratoire du patient, dans le temps. Il existe différentes méthodes permettant d'obtenir cette courbe à une dimension. De telles méthodes sont considérées comme connues de l'homme du métier.

On peut notamment citer la méthode qui consiste à considérer que le mouvement de la référence patient est vertical et à ne considérer que l'axe X de la caméra, même s'il n'est pas perpendiculaire par rapport au sol. Cependant, dans ce cas, une partie de l'amplitude du mouvement de la référence patient sera perdue.

On peut également citer la méthode, plus précise, qui consiste à effectuer une analyse en composantes principales des positions de la référence patient. Ces positions sont analysées par rapport à l'axe principal du mouvement, dénommé composante principale (illustré en pointillé sur la figure 4, pour exemple), qui correspond à l'axe sur lequel s'effectue le mouvement respiratoire. Les positions de la référence patient sont affichées selon cette composante principale. Une telle méthode permet de s'affranchir des éventuels mouvements de la caméra pouvant générer une modification des coordonnées de la référence patient, même lorsque le mouvement respiratoire du patient est identique.

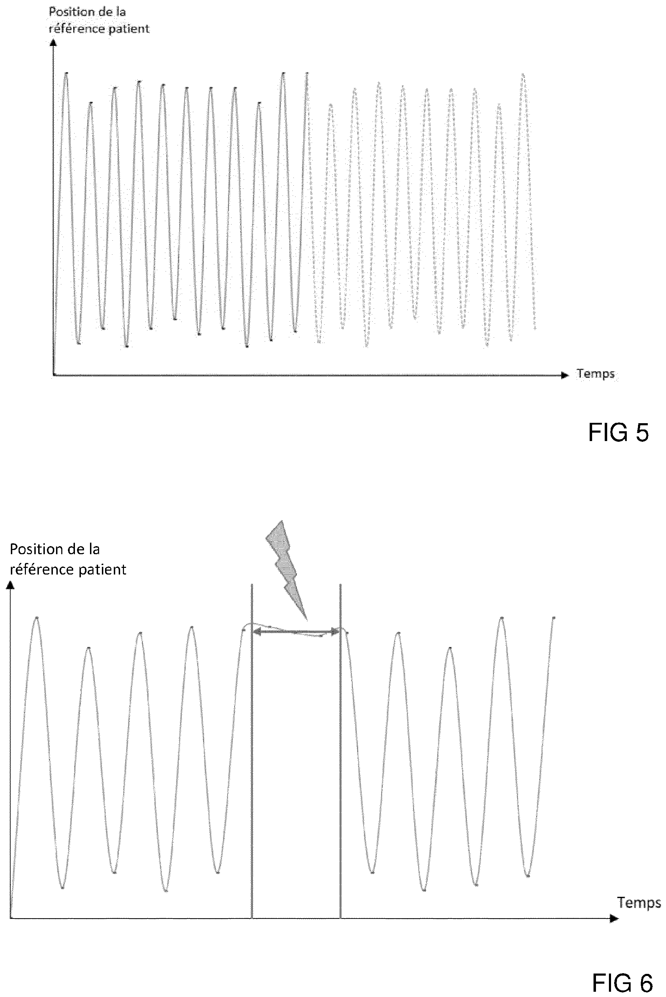

La courbe en trait continu sur la figure 5 représente un exemple de représentation des mouvements de la référence patient, donc une représentation du cycle respiratoire du patient, dans le temps.

Cette courbe peut être affichée sur l'équipement d'interface homme-machine 32. Simultanément, l'unité de contrôle, d'autre part, reçoit en continu une information du détecteur 13 de rayons X.

Lorsque la référence patient 10 comporte un scintillateur, l'unité de contrôle reçoit, également de la caméra, des données relatives à la présence ou l'absence de rayons X. L'unité de contrôle 30 détermine les positions de la référence patient pour lesquelles des rayons X ont été détectés, et enregistre ces positions dans son module mémoire 31.

Lorsque la référence patient 10 comporte un dosimètre, l'unité de contrôle reçoit, dudit dosimètre, via le moyen de communication filaire, des données relatives à la dose de rayons X qu'il a reçu. L'unité de contrôle 30 détermine les positions de la référence patient pour lesquelles la dose de rayons X est supérieure à 0, strictement, et enregistre ces positions dans son module mémoire 31.

Les positions enregistrées dans le module mémoire 31 correspondent aux positions pendant la durée de l'exposition aux rayons X, c'est-à-dire pendant l'acquisition de l'image médicale.

Pendant la durée de l'exposition aux rayons X, que ce soit dans le cas où la respiration du patient est bloquée ou dans le cas où la respiration du patient n'est pas bloquée, plusieurs positions de la référence patient sont généralement détectées. Ces positions ne sont pas nécessairement identiques, mais sont très proches les unes des autres, comme illustré sur la figure 6. Ces positions sont d'autant plus proches les unes des autres que la respiration du patient est bloquée.

Pour obtenir une position cible de la référence patient lors de l'acquisition de l'image médicale, une solution consiste à réaliser une moyenne desdites positions déterminées pendant la durée d'exposition aux rayons X. Cette solution permet de s'affranchir avantageusement du bruit de mesure de la caméra.

Une autre solution, applicable uniquement lorsque la référence patient comporte un dosimètre, consisterait à réaliser une moyenne desdites positions déterminées pendant la durée d'exposition aux rayons X, pondérée par la dose de rayons X reçue à chaque position.

Une autre solution consisterait à réaliser un médian desdites positions déterminées pendant la durée d'exposition aux rayons X. Cette solution permet de s'affranchir avantageusement d'une position aberrante.

Une autre solution consisterait, à partir de la connaissance a priori de la courbe de dose reçue au cours du temps par un objet ponctuel lors de l'acquisition d'images médicales, de déterminer la ou les positions de la référence patient lors d'une phase spécifique de l'exposition. Cette solution permet de sélectionner avec précision les positions de la référence patient lors des phases de l'exposition ayant la plus grosse contribution à la formation des images médicales.

À l'issue de cette troisième étape 102, la position cible de la référence patient 10 lors de l'acquisition de l'image médicale intra-opératoire est déterminée.

Le mouvement de la référence patient 10 étant équivalent au mouvement du cycle respiratoire du patient, la position cible de la référence patient 10 correspond à une phase dite cible du cycle respiratoire dans le temps.

L'opérateur peut à présent planifier l'intervention médicale, par exemple l'insertion d'une aiguille dans le corps du patient, à partir d'une image médicale. Dans un mode de mise en œuvre, l'opérateur réalise lui-même le geste médical d'insertion.

Dans un autre mode de mise en œuvre, l'opérateur est assisté par le robot médical 50 et le geste médical est réalisé par ledit robot médical. Le robot médical 50 est alors configuré en fonction d'un plan d'intervention qui sera mémorisé dans le module mémoire 31 de l'unité de contrôle 30. Le robot médical se positionne, par rapport au patient, conformément au plan d'intervention et grâce au système de navigation optique.

Le plan d'intervention est généré à partir d'une image médicale.

Dans un exemple de mise en œuvre, le plan d'intervention est généré à partir de l'image médicale intra-opératoire acquise par le dispositif d'imagerie 40 par rayons X, lors de la deuxième étape 101 du procédé.

Dans un autre exemple de mise en œuvre, le plan d'intervention est généré à partir d'une image médicale, dite pré-opératoire. Cette image médicale préopératoire a été réalisée avant l'intervention médicale, par exemple plusieurs jours ou plusieurs heures avant l'intervention médicale. Dans ce cas, la position réelle de l'anatomie d'intérêt du patient au moment de l'intervention ne correspond pas nécessairement à une position prévue ou modélisée pendant une phase de planification préopératoire. Il est donc avantageux de pouvoir recaler une image préopératoire à partir de laquelle une action à réaliser sur la zone anatomique cible est planifiée avec une image intra-opératoire représentant précisément la position de la zone anatomique cible du patient 1 au moment de l'intervention.

Il existe différentes méthodes de recalage d'une image avec une autre. De telles méthodes sont considérées comme connues de l'homme du métier.

Dans une quatrième étape 103, une position, dite candidate, de la référence patient 10 est déterminée.

Dans un premier mode de mise en œuvre de cette quatrième étape, la détermination de la position candidate est réalisée pendant un blocage temporaire de la respiration du patient.

Dans une première phase de ce premier mode de mise en œuvre, la respiration du patient est à nouveau bloquée. La respiration doit préférentiellement être bloquée à la même phase du cycle respiratoire que lors de l'acquisition de l'image médicale.

Dans un exemple de mise en œuvre de cette première phase, pour décider du moment du blocage de la respiration du patient, lorsque le patient est sous anesthésie générale par exemple, l'opérateur peut se fier par exemple à la position du soufflet du respirateur du système d'anesthésie, en positionnant le soufflet sensiblement à la même position que la position du soufflet lors du blocage de la respiration pour l'acquisition de l'image médicale.

Dans un autre exemple de mise en œuvre de cette première phase, on peut avoir recours à un modèle de prédiction du cycle respiratoire du patient. A partir de l'enregistrement des mouvements de la référence patient dans le temps, donc du mouvement respiratoire du patient, il est possible d'estimer un modèle de prédiction du cycle respiratoire. Ce modèle de prédiction est déterminé par l'unité de contrôle 30.

La figure 5 illustre, en trait discontinu, les positions prédites dans le temps de la référence patient, donc le cycle respiratoire prédit du patient.

A partir de cette prédiction, il est alors possible d'anticiper l'instant où la respiration du patient doit être bloquée.

L'unité de contrôle 30 peut transmettre une information à l'opérateur, par exemple sous la forme d'un signal sonore ou visuel, lui signalant qu'il peut bloquer la respiration du patient.

Dans une seconde phase du premier mode de mise en œuvre, après blocage de la respiration du patient, la position candidate de la référence patient 10 est déterminée.

Cette position candidate est déterminée à partir des coordonnées de la référence patient acquises par la caméra, comme expliqué lors de la troisième étape 102 du procédé.

Dans un deuxième mode de mise en œuvre de la quatrième étape, la détermination de la position candidate est réalisée sans blocage, même temporaire, de la respiration du patient.

Dans un exemple de mise en œuvre de ce deuxième mode, on peut avoir recours à un modèle de prédiction du cycle respiratoire du patient. A partir de l'enregistrement des mouvements de la référence patient dans le temps, donc du mouvement respiratoire du patient, il est possible d'estimer un modèle de prédiction du cycle respiratoire. Ce modèle de prédiction est déterminé par l'unité de contrôle 30.

A partir de cette prédiction, il est alors possible d'anticiper l'instant où la référence patient est proche de la position cible.

La position candidate de la référence patient 10 est alors déterminée. Cette position candidate est déterminée à partir des coordonnées de la référence patient acquises par la caméra, comme expliqué lors de la troisième étape 102 du procédé

Dans une cinquième étape 104, la position candidate de la référence patient 10 est comparée à la position cible.

La comparaison de la position candidate avec la position cible, enregistrée dans le module mémoire 31, est réalisée par l'unité de contrôle.

### Cas où la position candidate est déterminée pendant le blocage de la respiration du patient (premier mode de mise en œuvre de la quatrième étape)

Lorsque la position candidate de la référence patient 10 se situe dans une plage de tolérance prédéfinie par rapport à la position cible, on considère que la respiration du patient a été bloquée à un instant opportun, c'est à dire à la même phase du cycle respiratoire que lors de l'acquisition de l'image médicale. La plage de tolérance est, de préférence, de ± 10% de la position cible, plus préférentiellement, de ± 5% de la position cible. Une information est alors transmise pour signaler que l'intervention peut commencer, c'est-à-dire que le moment est optimal pour pratiquer le geste médical, tel que par exemple l'insertion d'un instrument médical vers la zone anatomique cible du patient.

Dans un exemple de mise en œuvre, l'information est transmise à l'opérateur. Cette information peut être transmise par exemple sous la forme d'un signal sonore via le voyant lumineux du dispositif de synchronisation, et/ou d'un signal visuel via l'alarme du dispositif de synchronisation, sur l'équipement d'interface homme-machine 32 et/ou via un voyant lumineux, type DEL, tel que par exemple celui du robot médical, lorsque l'opérateur est assisté par ledit robot médical. Le voyant lumineux du dispositif de synchronisation et/ou du robot médical peut par exemple émettre une lumière de couleur fixe.

Dans un autre exemple de mise en œuvre, lorsque l'opérateur est assisté par le robot médical 50, l'information est transmise directement au robot médical. Le geste médical est automatiquement déclenché et pratiqué par le robot médical. Lorsque l'intervention médicale est terminée, la respiration du patient est débloquée.

Lorsque la position candidate de la référence patient 10 ne se situe pas dans la plage de tolérance prédéfinie par rapport à la position cible, on considère que la respiration du patient n'a pas été bloquée à la même phase du cycle respiratoire que lors de l'acquisition de l'image médicale. Une information est alors transmise à l'opérateur pour lui signaler de ne pas commencer l'intervention, c'est-à-dire que le moment n'est pas optimal pour pratiquer le geste médical. Cette information peut être transmise par exemple sous la forme d'un signal sonore via l'alarme du dispositif de synchronisation, ou encore d'un signal visuel via le voyant lumineux du dispositif de synchronisation, sur l'équipement d'interface homme-machine 32 ou via le voyant lumineux, type DEL, tel que par exemple celui du robot médical, lorsque l'opérateur est assisté par ledit robot médical. Le voyant lumineux du dispositif de synchronisation et/ou du robot médical peut par exemple émettre une lumière de couleur clignotante.

La respiration du patient est alors débloquée.

Les étapes 103 et 104 sont réitérées jusqu'à ce que la position candidate de la référence patient 10 se situe dans une plage de tolérance prédéfinie par rapport à la position cible.

Lorsque l'intervention médicale est terminée, la respiration du patient est débloquée.

### Cas où la position candidate est déterminée sans blocage de la respiration du patient (deuxième mode de mise en œuvre de la quatrième étape)

Lorsque la position candidate de la référence patient 10 se situe dans une plage de tolérance prédéfinie par rapport à la position cible, on considère que la phase du cycle respiratoire du patient est proche la même phase du cycle respiratoire que lors de l'acquisition de l'image médicale. La plage de tolérance est, par exemple, comprise entre ± 10% de la position cible, de préférence comprise entre ± 5% de la position cible, ou encore de préférence comprise - 10% et - 5% de la position cible. Une information est alors transmise pour signaler que l'intervention peut commencer, c'est-à-dire que le moment est optimal pour pratiquer le geste médical, tel que par exemple l'insertion d'un instrument médical vers la zone anatomique cible du patient.

Dans un exemple de mise en œuvre, l'information est transmise à l'opérateur. Cette information peut être transmise par exemple sous la forme d'un signal sonore via le voyant lumineux du dispositif de synchronisation, et/ou d'un signal visuel via l'alarme du dispositif de synchronisation, sur l'équipement d'interface homme-machine 32 et/ou via un voyant lumineux, type DEL, tel que par exemple celui du robot médical, lorsque l'opérateur est assisté par ledit robot médical. Le voyant lumineux du dispositif de synchronisation et/ou du robot médical peut par exemple émettre une lumière de couleur fixe.

Dans un autre exemple de mise en œuvre, lorsque l'opérateur est assisté par le robot médical 50, l'information est transmise directement au robot médical. Le geste médical est automatiquement déclenché et pratiqué par le robot médical.

Lorsque la position candidate de la référence patient 10 ne se situe pas dans la plage de tolérance prédéfinie par rapport à la position cible, on considère que la phase du cycle respiratoire du patient est proche, et en amont, de la même phase du cycle respiratoire que lors de l'acquisition de l'image médicale. Une information est alors transmise à l'opérateur pour lui signaler de ne pas commencer l'intervention, c'est-à-dire que le moment n'est pas optimal pour pratiquer le geste médical. Cette information peut être transmise par exemple sous la forme d'un signal sonore via l'alarme du dispositif de synchronisation, ou encore d'un signal visuel via le voyant lumineux du dispositif de synchronisation, sur l'équipement d'interface homme-machine 32 ou via le voyant lumineux, type DEL, tel que par exemple celui du robot médical, lorsque l'opérateur est assisté par ledit robot médical. Le voyant lumineux du dispositif de synchronisation et/ou du robot médical peut par exemple émettre une lumière de couleur clignotante. Les étapes 103 et 104 sont réitérées jusqu'à ce que la position candidate de la référence patient 10 se situe dans une plage de tolérance prédéfinie par rapport à la position cible, toujours sans blocage de la respiration.

Les modes de mise en œuvre et de réalisation du procédé et du dispositif de synchronisation considérés ci-dessus ont été décrits à titre d'exemples non limitatifs, et d'autres variantes sont par conséquent envisageables.

Notamment, le procédé a été décrit dans le cas d'un dispositif de localisation optique, en l'occurrence une caméra. Le procédé peut s'appliquer, par analogie, au dispositif de localisation électromagnétique, sans se départir du cadre de l'invention.

La description ci-avant illustre clairement que, par ses différentes caractéristiques et leurs avantages, la présente invention atteint les objectifs fixés. En particulier, le procédé permet de détecter l'instant exact du cycle respiratoire du patient lors de l'acquisition de l'image médicale et de déterminer l'instant optimal du cycle respiratoire pour assister l'opérateur à pratiquer le geste médical.

## Revendications

1. Dispositif de synchronisation pour la détermination d'un instant du cycle respiratoire d'un patient pour assister une intervention médicale sur ledit patient, comportant :
- un dispositif de localisation (20),
- une référence patient (10), destinée à être positionnée sur le corps du patient (1), et comportant des marqueurs radio-opaques (11), au moins un élément de localisation (12) configuré pour être détectable par le dispositif de localisation (20), et un détecteur (13) de rayons X destiné à coopérer avec un dispositif d'imagerie (40) par rayons X,
- une unité de contrôle (30) pour l'enregistrement et le traitement des données provenant du dispositif de localisation et de la référence patient,
dans lequel le dispositif de localisation (20) est configuré pour enregistrer, en continu, des mouvements de la référence patient (10), les mouvements de la référence patient correspondant au cycle respiratoire du patient, et pour transmettre à l'unité de contrôle (30) les données relatives aux mouvements de la référence patient, en ce que le détecteur (13) de rayons X est configuré pour détecter le moment où une image médicale est acquise par le dispositif d'imagerie (40) par rayons X et pour transmettre les données correspondantes à l'unité de contrôle (30) et dans lequel l'unité de contrôle (30) est configurée pour :
∘ déterminer une position, dite cible, de la référence patient (10), dans un repère du dispositif de localisation (20), à partir du au moins un élément de localisation (12) et du détecteur (13) de rayons X positionnés sur la référence patient (10),
∘ déterminer une position, dite candidate, de la référence patient (10), dans le repère du dispositif de localisation (20), à partir du au moins un élément de localisation (12) positionné sur la référence patient (10),
∘ comparer la position candidate et la position cible,
o lorsque la position candidate ne se situe pas dans une plage de tolérance prédéfinie par rapport à la position cible, réitérer les étapes de détermination de la position candidate et de comparaison des positions cible et candidate jusqu'à ce que la position candidate se situe dans une plage de tolérance prédéfinie par rapport à la position cible.

2. Dispositif de synchronisation selon la revendication 1 dans lequel le dispositif de localisation (20) est un dispositif de localisation optique et dans lequel la référence patient (10) comporte au moins trois éléments de localisation optiques.

3. Dispositif de synchronisation selon la revendication 1, dans lequel le dispositif de localisation (20) est un dispositif de localisation électromagnétique et dans lequel la référence patient (10) comporte au moins un élément de localisation électromagnétique.

4. Dispositif de synchronisation selon l'une des revendications 1 à 3, dans lequel le détecteur (13) de rayons X est un dosimètre ou un scintillateur.

5. Dispositif de synchronisation selon la revendication 1 dans lequel l'unité de contrôle (30) est configurée pour déterminer, à partir de l'ensemble des positions prises par la référence patient pendant la durée de l'exposition aux rayons X, la position cible de la référence patient.

6. Dispositif de synchronisation selon l'une des revendications 1 à 5 dans lequel l'unité de contrôle (30) est configurée pour estimer, à partir d'un modèle de prédiction du cycle respiratoire du patient, l'instant où la référence patient est proche de la position cible, lors de la détermination d'une position candidate.

7. Dispositif de synchronisation selon l'une des revendications 1 à 6 comportant un équipement d'interface homme-machine (32) configuré pour informer un opérateur, sous la forme d'un signal sonore et/ou d'un signal visuel, du résultat de la comparaison entre la position candidate et la position cible.

8. Dispositif de synchronisation selon la revendication 7 dans lequel l'équipement d'interface homme-machine (32) est un écran de visualisation.

9. Dispositif de synchronisation selon l'une des revendications 1 à 8 dans lequel l'unité de contrôle (30) est configurée pour planifier une intervention assistée par un robot médical entre les étapes de détermination de la position cible et de la position candidate.

10. Ensemble comportant un robot médical (50) et un dispositif de synchronisation selon l'une des revendications 1 à 9, le robot médical comportant une base, un bras articulé (51) dont une extrémité est reliée à la base, et une unité de contrôle.

11. Ensemble selon la revendication 10 dans lequel l'unité de contrôle du robot médical et l'unité de contrôle (30) du dispositif de synchronisation forment une seule et même unité de contrôle.

12. Ensemble selon l'une des revendications 10 ou 11 dans lequel le robot médical (50) comporte un équipement d'interface homme-machine.

13. Procédé de détermination d'un instant du cycle respiratoire d'un patient (1) pour assister une intervention médicale sur ledit patient mis en œuvre par une unité de contrôle (30), comportant les étapes de :
100 - enregistrement, en continu, des mouvements d'une référence patient (10) placée à proximité d'une zone anatomique cible du patient (1), les mouvements de la référence patient correspondant au cycle respiratoire du patient,
101 - acquisition, par un dispositif d'imagerie (40) par rayons X, d'une image médicale de ladite zone anatomique cible du patient,
102 - détermination d'une position, dite cible, de la référence patient (10) lors de l'acquisition de l'image médicale, à partir d'au moins un élément de localisation (12) positionné sur la référence patient (10) configuré pour être détectable par un dispositif de localisation (20) et à partir d'un détecteur (13) de rayons X positionné également sur la référence patient (10) et configuré pour détecter le moment où une image médicale est acquise par le dispositif d'imagerie (40) par rayons X et pour transmettre les données correspondantes à l'unité de contrôle (30),
103 - détermination d'une position, dite candidate, de la référence patient (10), à partir du au moins un élément de localisation (12) positionné sur la référence patient (10),
104 - comparaison de la position candidate et de la position cible.

14. Procédé selon la revendication 13, dans lequel la position cible de la référence patient (10) est déterminée à partir de l'ensemble des positions prises par la référence patient (10) pendant la durée de l'exposition aux rayons X.

15. Procédé selon l'une des revendications 13 ou 14 dans lequel le moment où la position candidate se situe dans une plage de tolérance prédéfinie par rapport à la position cible est estimé à partir d'un modèle de prédiction du cycle respiratoire du patient.

16. Procédé selon l'une des revendications 13 à 15 selon lequel un signal sonore et/ou un signal visuel est généré lorsque la position candidate se situe dans une plage de tolérance prédéfinie par rapport à la position cible.

17. Procédé selon l'une des revendications 13 à 16 comportant une étape de planification d'une intervention assistée par un robot médical entre les étapes de détermination de la position cible et de la position candidate.

## Patentansprüche

1. Synchronisationsvorrichtung für die Bestimmung eines Zeitpunkts des Atemzyklus eines Patienten zum Unterstützen eines medizinischen Eingriffs an dem Patienten, umfassend:
- eine Lokalisierungsvorrichtung (20),
- eine Patientenreferenz (10), die dafür bestimmt ist, auf dem Körper (1) des Patienten positioniert zu werden, und strahlenundurchlässige Marker (11), mindestens ein Lokalisierungselement (12), das konfiguriert ist, um durch die Lokalisierungsvorrichtung (20) erkennbar zu sein, und einen Röntgendetektor (13) umfasst, der dafür bestimmt ist, mit einer Röntgenbildgebungsvorrichtung (40) zusammenzuarbeiten,
- eine Steuereinheit (30) für das Aufzeichnen und Verarbeiten von Daten, die von der Lokalisierungsvorrichtung und der Patientenreferenz stammen,
wobei die Lokalisierungsvorrichtung (20) konfiguriert ist, um Bewegungen der Patientenreferenz (10) kontinuierlich aufzuzeichnen, wobei die Bewegungen der Patientenreferenz dem Atemzyklus des Patienten entsprechen, und um Daten an die Steuereinheit (30) in Bezug auf die Bewegungen der Patientenreferenz zu übermitteln, dass der Röntgendetektor (13) konfiguriert ist, um den Moment zu erkennen, in dem ein medizinisches Bild durch die Röntgenbildgebungsvorrichtung (40) erfasst wird, und um die entsprechenden Daten an die Steuereinheit (30) zu übermitteln, und wobei die Steuereinheit (30) konfiguriert ist zum:
o Bestimmen einer als Ziel bezeichneten Position der Patientenreferenz (10) in einer Markierung der Lokalisierungsvorrichtung (20), von dem mindestens einen Lokalisierungselement (12) und dem an der Patientenreferenz (10) positionierten Röntgendetektor (13),
o Bestimmen einer als Kandidat bezeichneten Position der Patientenreferenz (10) in der Markierung der Lokalisierungsvorrichtung (20), von dem mindestens einen Lokalisierungselement (12), das an der Patientenreferenz (10) positioniert ist,
∘ Vergleichen der Kandidatenposition und der Zielposition,
o wenn die Kandidatenposition nicht in einem vordefinierten Toleranzbereich relativ zu der Zielposition liegt, Wiederholen der Schritte des Bestimmens der Kandidatenposition und des Vergleichens der Ziel- und der Kandidatenposition, bis die Kandidatenposition in einem vordefinierten Toleranzbereich relativ zu der Zielposition liegt.

2. Synchronisationsvorrichtung nach Anspruch 1, wobei die Lokalisierungsvorrichtung (20) eine optische Lokalisierungsvorrichtung ist und wobei die Patientenreferenz (10) mindestens drei optische Lokalisierungselemente umfasst.

3. Synchronisationsvorrichtung nach Anspruch 1, wobei die Lokalisierungsvorrichtung (20) eine elektromagnetische Lokalisierungsvorrichtung ist und wobei die Patientenreferenz (10) mindestens ein elektromagnetisches Lokalisierungselement umfasst.

4. Synchronisationsvorrichtung nach einem der Ansprüche 1 bis 3, wobei der Röntgendetektor (13) ein Dosimeter oder ein Szintillator ist.

5. Synchronisationsvorrichtung nach Anspruch 1, wobei die Steuereinheit (30) konfiguriert ist, um von der Anordnung der Positionen, die die Patientenreferenz während der Dauer der Röntgenbestrahlung einnimmt, die Zielposition der Patientenreferenz zu bestimmen.

6. Synchronisationsvorrichtung nach einem der Ansprüche 1 bis 5, wobei die Steuereinheit (30) konfiguriert ist, um während der Bestimmung einer Kandidatenposition von einem Modell zum Vorhersagen des Atemzyklus des Patienten den Zeitpunkt zu schätzen, in dem die Patientenreferenz nahe an der Zielposition liegt.

7. Synchronisierungsvorrichtung nach einem der Ansprüche 1 bis 6, umfassend eine Mensch-Maschine-Schnittstellenausrüstung (32), die konfiguriert ist, um einen Bediener in Form eines akustischen Signals und/oder eines visuellen Signals über das Ergebnis des Vergleichs zwischen der Kandidatenposition und der Zielposition zu informieren.

8. Synchronisationsvorrichtung nach Anspruch 7, wobei die Mensch-Maschine-Schnittstellenausrüstung (32) ein Anzeigegerät ist.

9. Synchronisationsvorrichtung nach einem der Ansprüche 1 bis 8, wobei die Steuereinheit (30) konfiguriert ist, um zwischen den Schritten des Bestimmens der Zielposition und der Kandidatenposition einen durch einen medizinischen Roboter unterstützten Eingriff zu planen.

10. Anordnung, umfassend einen medizinischen Roboter (50) und eine Synchronisationsvorrichtung nach einem der Ansprüche 1 bis 9, wobei der medizinische Roboter eine Basis, einen Gelenkarm (51), dessen eines Ende mit der Basis verbunden ist, und eine Steuereinheit umfasst.

11. Anordnung nach Anspruch 10, wobei die Steuereinheit des medizinischen Roboters und die Steuereinheit (30) der Synchronisationsvorrichtung eine einzige Steuereinheit ausbilden.

12. Anordnung nach einem der Ansprüche 10 oder 11, wobei der medizinische Roboter (50) eine Mensch-Maschine-Schnittstellenausrüstung umfasst.

13. Verfahren zum Bestimmen eines Zeitpunkts des Atemzyklus eines Patienten (1), um einen durch eine Steuereinheit (30) durchgeführten medizinischen Eingriff an dem Patienten zu unterstützen, umfassend die Schritte:
100 - kontinuierliches Aufzeichnen der Bewegungen einer Patientenreferenz (10), die in der Nähe eines anatomischen Zielbereichs des Patienten (1) platziert ist, wobei die Bewegungen der Patientenreferenz dem Atemzyklus des Patienten entsprechen,
101 - Erfassen eines medizinischen Bilds des anatomischen Zielbereichs des Patienten durch eine Röntgenbildgebungsvorrichtung (40),
102 - Bestimmen einer als Ziel bezeichneten Position der Patientenreferenz (10)
während der Erfassung des medizinischen Bilds von mindestens einem Lokalisierungselement (12), das auf der Patientenreferenz (10) positioniert ist, das konfiguriert ist, um durch eine Lokalisierungsvorrichtung (20) erfassbar zu sein, und von einem Röntgendetektor (13), der ebenfalls auf der Patientenreferenz (10) positioniert und konfiguriert ist, um den Moment zu erkennen, in dem ein medizinisches Bild durch die Röntgenbildgebungsvorrichtung (40) erfasst wird, und um die entsprechenden Daten an die Steuereinheit (30) zu übermitteln,
103 - Bestimmen einer als Kandidat bezeichneten Position der Patientenreferenz (10) von dem mindestens einen Lokalisierungselement (12), das an der Patientenreferenz (10) positioniert ist,
104 - Vergleichen der Kandidatenposition und der Zielposition.

14. Verfahren nach Anspruch 13, wobei die Zielposition der Patientenreferenz (10) von der Anordnung der Positionen bestimmt wird, die die Patientenreferenz (10) während der Dauer der Röntgenbestrahlung einnimmt.

15. Verfahren nach einem der Ansprüche 13 oder 14, wobei der Moment, zu dem die Kandidatenposition in einem vordefinierten Toleranzbereich relativ zu der Zielposition liegt, von einem Modell zum Vorhersagen des Atemzyklus des Patienten geschätzt wird.

16. Verfahren nach einem der Ansprüche 13 bis 15, wobei ein akustisches Signal und/oder ein optisches Signal erzeugt wird, wenn die Kandidatenposition relativ zu der Zielposition in einem vordefinierten Toleranzbereich liegt.

17. Verfahren nach einem der Ansprüche 13 bis 16, umfassend einen Schritt des Planens eines durch einen medizinischen Roboter unterstützten Eingriffs zwischen den Schritten des Bestimmens der Zielposition und der Kandidatenposition.

## Claims

1. Synchronization device for determining a moment of the respiratory cycle of a patient in order to assist a medical intervention on said patient, comprising:
- a locating device (20),
- a patient reference (10), intended to be positioned on the body of the patient (1), and comprising radiopaque markers (11), at least one locating element (12) configured to be detectable by the locating device (20), and an X-ray detector (13) intended to cooperate with an X-ray imaging device (40),
- a control unit (30) for recording and processing data from the locating device and the patient reference,
wherein the locating device (20) is configured to continuously record the movements of the patient reference (10), the movements of the patient reference corresponding to the patient's respiratory cycle, and to transmit data relating to the movements of the patient reference to the control unit (30), in that the X-ray detector (13) is configured to detect the moment when a medical image is acquired by the X-ray imaging device (40) and to transmit the corresponding data to the control unit (30) and wherein the control unit (30) is configured to:
o determine a position, referred to as a target position, of the patient reference (10) in a reference frame of the locating device (20) from the at least one locating element (12) and the X-ray detector (13) which are positioned on the patient reference (10),
o determine a position, referred to as a candidate position, of the patient reference (10) in the reference frame of the locating device (20) from the at least one locating element (12) which is positioned on the patient reference (10),
o compare the candidate position and the target position,
o when the candidate position is not within a predefined tolerance range relative to the target position, repeat the steps of determining the candidate position and comparing the target and candidate positions until the candidate position is within a predefined tolerance range relative to the target position.

2. Synchronization device according to claim 1, wherein the locating device (20) is an optical locating device and wherein the patient reference (10) comprises at least three optical locating elements.

3. Synchronization device according to claim 1, wherein the locating device (20) is an electromagnetic locating device and wherein the patient reference (10) comprises at least one electromagnetic locating element.

4. Synchronization device according to one of claims 1 to 3, wherein the X-ray detector (13) is a dosimeter or a scintillator.

5. Synchronization device according to claim 1, wherein the control unit (30) is configured to determine the target position of the patient reference from all the positions taken by the patient reference during the period of exposure to X-rays.

6. Synchronization device according to one of claims 1 to 5, wherein the control unit (30) is configured to estimate, on the basis of a model for predicting the patient's respiratory cycle, the moment when the patient reference is close to the target position, when determining a candidate position.

7. Synchronization device according to one of claims 1 to 6, comprising a piece of human-machine interface equipment (32) which is configured to inform an operator, in the form of an audible signal and/or a visual signal, of the result of the comparison between the candidate position and the target position.

8. Synchronization device according to claim 7, wherein the piece of human-machine interface equipment (32) is a display screen.

9. Synchronization device according to one of claims 1 to 8, wherein the control unit (30) is configured to plan a medical robot-assisted intervention between the steps of determining the target position and the candidate position.

10. Assembly comprising a medical robot (50) and a synchronization device according to one of claims 1 to 9, the medical robot comprising a base, an articulated arm (51), one end of which is connected to the base, and a control unit.

11. Assembly according to claim 10, wherein the control unit of the medical robot and the control unit (30) of the synchronization device form a single control unit.

12. Assembly according to one of claims 10 or 11, wherein the medical robot (50) comprises a piece of human-machine interface equipment.

13. Method for determining a moment of the respiratory cycle of a patient (1) in order to assist a medical intervention on said patient implemented by a control unit (30), comprising the steps of:
100 - continuously recording the movements of a patient reference (10) placed close to a target anatomical zone of the patient (1), the movements of the patient reference corresponding to the patient's respiratory cycle,
101 - acquiring, by means of an X-ray imaging device (40), a medical image of said target anatomical zone of the patient,
102 - determining a so-called target position of the patient reference (10) when acquiring the medical image, from at least one locating element (12) positioned on the patient reference (10), which is configured to be detectable by a locating device (20) and from an X-ray detector (13) also positioned on the patient reference (10) and which is configured to detect the moment when a medical image is acquired by the X-ray imaging device (40) and to transmit the corresponding data to the control unit (30),
103 - determining a so-called candidate position of the patient reference (10) from the at least one locating element (12) positioned on the patient reference (10),
104 - comparing the candidate position and the target position.

14. Method according to claim 13, wherein the target position of the patient reference (10) is determined from all the positions taken by the patient reference (10) during the period of exposure to X-rays.

15. Method according to one of claims 13 or 14, wherein the moment at which the candidate position is within a predefined tolerance range relative to the target position is estimated on the basis of a model for predicting the patient's respiratory cycle.

16. Method according to one of claims 13 to 15, whereby an audible signal and/or a visual signal is generated when the candidate position is within a predefined tolerance range relative to the target position.

17. Method according to one of claims 13 to 16, comprising a step of planning a medical robot-assisted intervention between the steps of determining the target position and the candidate position.
